# EUROPEAN PATENT SPECIFICATION

(11) **EP 3 030 132 B1**
(45) Date of publication and mention of the grant of the patent: **23.09.2020**
(21) Application number: 14715140.1
(22) Date of filing: 13.02.2014
(51) Int. Cl.: A61B 1/00, A61B 1/06, F21V 8/00, G02B 6/04, G02B 6/42, G02B 6/26, A61B 1/12, G02B 6/12

(54) **METHOD FOR PRODUCTION OF LOCAL ILLUMINATION PATCH CORD AND CORRESPONDING PATCH CORD**
VERFAHREN ZUR HERSTELLUNG EINES PATCHKABELS FÜR EINE LOKALE BELEUCHTUNG UND ENTSPRECHENDES PATCHKABEL
PROCÉDÉ DE PRODUCTION DE CORDON DE RACCORDEMENT LOCAL D'ÉCLAIRAGE ET CORDON DE RACCORDEMENT CORRESPONDANT

(30) Priority: 12.07.2013 PT 10706113
(43) Date of publication of application: 15.06.2016
(73) Proprietor: Famolde-Fabricação e Comercialização de Moldes SA, 2431-902 Marinha Grande (PT)
(72) Inventor: HENRIQUES MARTINS, Joaquim, 2430-067 Marinha Grande (PT); DINIS MARTINS, Bruno, 2430-108 Marinha Grande (PT); FERREIRA OLIVEIRA, Pedro Jorge, 2430-167 Marinha Grande (PT); DIAS, Marco Filipe, 2430-057 Marinha Grande (PT); DUARTE DE PINHO, Carlos Alberto, 2430-426 Marinha Grande (PT)
(74) Representative: Pereira da Cruz, Joao
(86) International application number: PCT/PT2014/000010
(87) International publication number: WO 2015/005813

(56) References cited:
- EP-A1- 0 025 969
- EP-A2- 1 772 096
- WO-A1-2008/037195
- WO-A1-2012/121318
- US-A1- 2012 243 836

## Description

### Technical field and state of the art of the invention

The scope of present invention relates to the field of optical illumination, with particular focus on the medical sector as auxiliary device in surgery.

In the current market there are numerous applications for optical fiber, but there are few available techniques for the design of fiber-optic cables for illumination. Among the various available cables, none meets such requirements as: flexibility, light efficiency and decrease of factors affecting its resistance, namely the heat.

In opposition to the current accessories available in the market, present invention decentralizes the thermal filter from the cable to the coupling, enabling heat dissipation before entering the illumination cable (1). Regarding the luminous efficiency, present invention defines a technique with a more effective manner of aggregating the multi-fibers of the cable (1), significantly saving production times, and raises polishing quality to the highest level of light transmission.

Patent document WO2008/037195A1 is considered to be the closest prior art of the present invention and it discloses a method for production of patch cords suitable for local illumination, but it doesn't refer to a thermal filter applied in coupling, followed by an O-ring said couplings being supplied separately from the patch cord.

### Brief description of the invention

Present invention reflects the need of having a product that incorporates a particular medical device and allowing it to have autonomous qualities of flexibility, reduced volume, high capacity of light transmission, longevity and low cost.

Since it was not found in the prior art a product with such requirements, present invention has been developed with a reduced transmission section, but still with a very high capacity of carrying light, equivalent to similar devices with twice the cross-sectional area. To this end, PMMA (Poly-methylmethacrylate) fibers where used in alternative to the existing glass and liquid fibers. In fact, the PMMA fibers provide a higher quality both at the structural level - they do not break so easily - and at the level of purity - a cooler white light - containing a low degree of impurities in its composition. A set of 19 fibers with a diameter of 0.0005m was aggregated and coated by a flexible crystal PVC sleeve (which in turn provides protection and flexibility to the patch cord), thus maximizing the flow of light in a reduced section of 0.0025m using a UV curing liquid adhesive.

This adhesive provides a very low curing time compared to an adhesive based on epoxy resin, and a very satisfactory fixation quality without damage of the fibers by either chemical reaction or by melting them through dry heat.

To ensure the quality of the transmitted light, and after testing several techniques including sandpaper of different grit sizes, diamond pastes and other polishing products existing on the market, it was found that the most effective method, fast and less expensive, would be the use of polishing soap. Therefore, after aggregation of the fibers the patch cord is submitted to a rectification process of the same (thus eliminating surplus) in a fine-grained grinding wheel (11), and subsequently in a felt buffing wheel (12) with a layer of soap, thus carrying out an action of wear and friction, respectively, at an averaged radial rotation.

In order to improve the longevity of the device, traditionally conditioned by fiber overheating and hence deterioration thereof, the thermal filter of the patch cord was decentralized to the coupling that accommodates the patch cord in each type/brand of light source commercially available.

### Description of the Figures

Figure 1 - Scheme of the Local Illumination Patch Cord.
Figure 2 - Module of Ultraviolet (UV) Exposure.
Figure 3 - Support of the Patch Cords for Exposure.
Figure 4 - Module of Fiber Polishing.

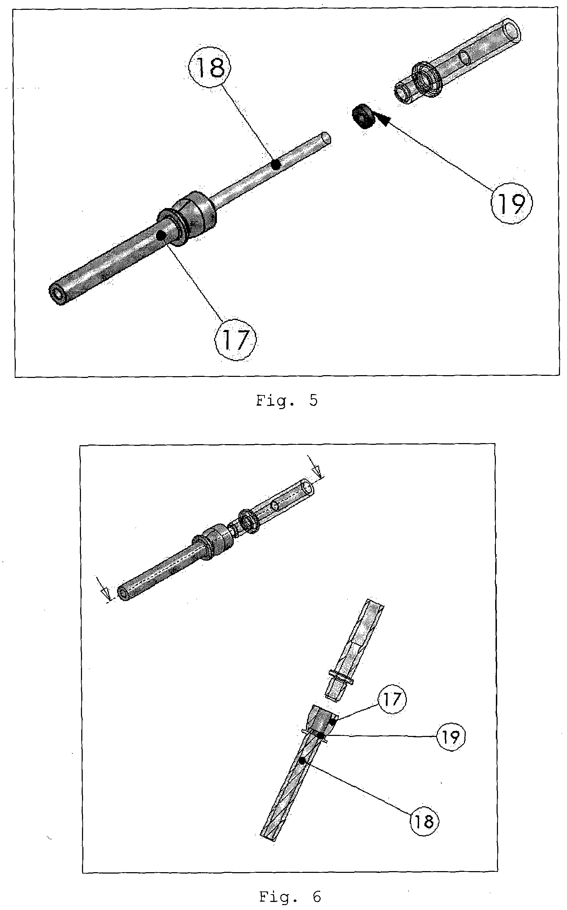

Figure 5 - Storz coupling with built-in thermal filter.
Figure 6 - Storz coupling with built-in thermal filter - cross-sectional view.

### List of reference numbers:

1. Multi-fiber optic cable (19×∅0.5), coated with flexible crystal PVC sleeve
2. 28mm tubular rivet
3. 88mm tubular rivet
4. ∅4 shrinkable sleeve
5. ∅9 shrinkable sleeve
6. Optic connector
7. UV lamp
8. Door
9. Spring clip;
10. Polishing machine
11. Fine-grained grinding wheel
12. Felt buffing wheel
13. Polishing support
14. Guide bar
15. Knob
16. Base of the polishing support sliding in the guide bar
17. Coupling
18. Thermal filter
19. O-ring

### Detailed Description of the Invention

Present invention discloses a method for producing a cable (1) for local illumination, which consists of a set of steps aided by the following modules:
- The ultrasonic module has the function to ensure the cleaning of metal components in order to maintain the final device free of residues;
- The Ultraviolet (UV) exposure module is intended to irradiate UV light, aiming the curing process in the liquid adhesion of the fibers to the metallic terminal;
- The polishing module has the functionality of performing the final polishing of the fiber surface, so that it is free from any influent light barrier. The module is semi-automatic and has two devices: one for rectification and another for final polishing;
- The thermal-sealing is a semi-automatic system which has the function of sealing the device packing with a sterilizable sealing paper.

The steps constituting present invention are:

### A. Preparation of cable terminals:

- Cleaning metallic parts: tubular rivets (2) (3) and optic connector (6) - this step should occur when receiving metallic parts, thereby allowing shortening of the illuminators production cycle time;
- Drying metallic parts - after the cleaning cycle and washing of the components with flowing water, metallic parts should be dried with compressed air;

### B. Cutting and installation of tubular rivets in the fiber:

- Cutting the cable, which must be cut into pieces of approximately 3m using a precision cutting pliers and should then be stripped in a portion of approximately 0.1m at one end and of 0.04m at the other end;
- Placing the metallic rivet with size 0.088m (3) in the 0.1m stripped end, with the respective head oriented to the cable side;
- Placing the metallic rivet with size 0.028m (2) in the other end, similarly to the aforementioned manner;
- Adjustment of each metallic rivet between the rivet head and the flexible PVC sleeve;

Note: the fibers should be aligned on the rivet, so they are not intertwined.

### C. Gluing - Application of UV adhesive and UV exposure

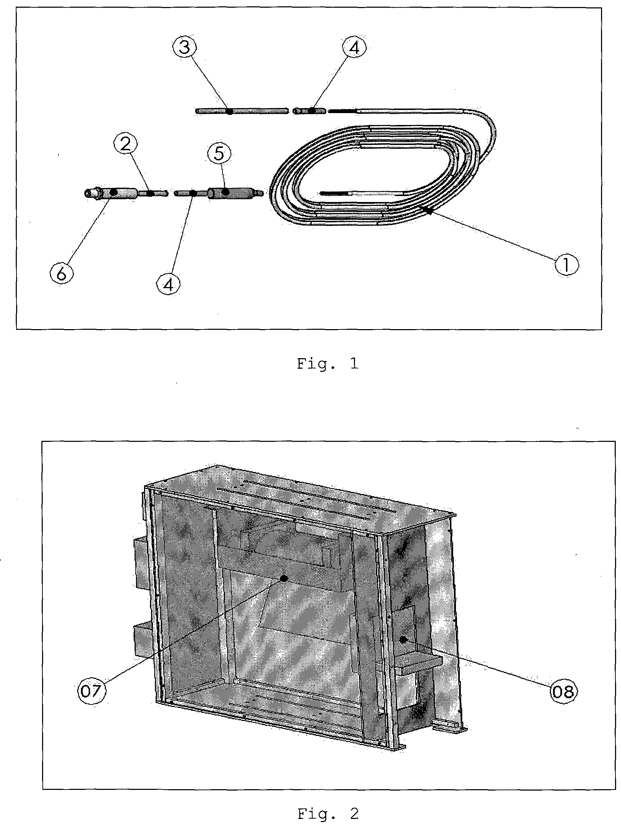

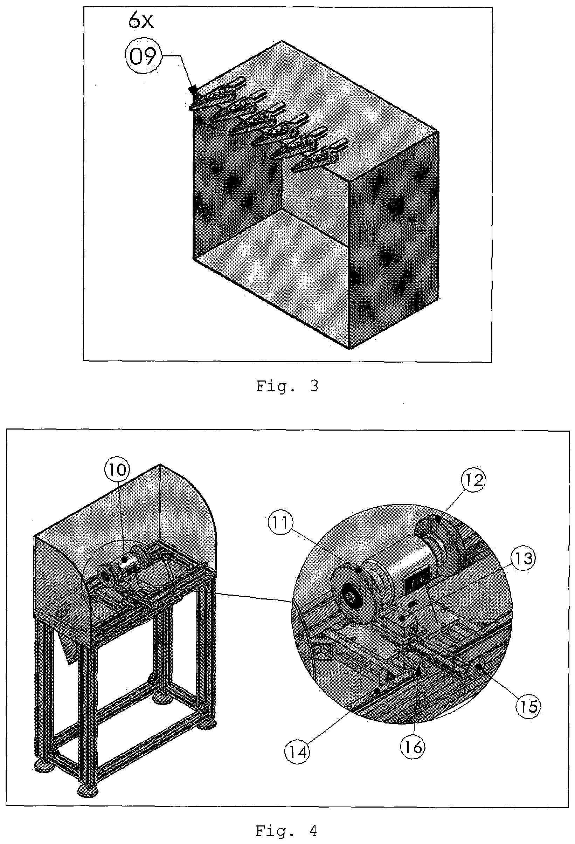

(this step takes place in the UV exposure module):
- Placing the patch cord with the two rivets in the UV exposure support, fixing them with the spring clips (9) ;
- Placing up to 3 patch cords in the support;
- Trimming to an average length of 0.01m the ends of each fiber passing the rivet head;
- Application on each end of the fiber (passing each rivet), with the UV adhesive applicator resting on the tip and pushing the plunger until leaving a small concave drop in the base of the fiber tip;
- UV exposure - UV exposure is made within the UV exposure module (Figure 2), in which exists a UV lamp (7) of approximately 1500W, opening the door (8) and placing the support (Figure 3) with the cables, whose tips are oriented upwards, during a predetermined time. This way, the production time is substantially reduced and hence the cost of the final product, which would not be possible when an epoxy adhesive or fusion method is used requiring curing and heating times of around 24 hours and 30 minutes, respectively.

### D. Fiber polishing

- The metallic tips of the patch cord should be placed in the support (13) of the polishing module;
- Adjusting the head of each rivet on the back surface of the support;
- Turning on the polishing machine (10) presenting the rectification and polishing wheels;
- Rectification of the tips with the fine-grained grinding wheel (11) while advancing the support by turning the knob (15);
- After rectification, sliding the support on the guide bar (14) to the felt buffing wheel (12) with soap along the base of the polishing support (16), and proceeding with polishing for about 30 seconds;
- Cleaning with a dry cloth;

### E. Quality Control:

- Cleaning the polished tips with gauze soaked in solvent (ethanol), followed by a dry gauze;
- Turning on the light source, connecting the patch cord to it and to the support of the luxmeter;
- Reading the amount of light (in lux);
   - if the reading does not show values above 300,000 lux, repeat the polishing procedure again;
   - if after 3 attempts the desired values are not obtained, reject the cable;

### F. Mounting of the optic connector and applicable optical device:

- Application of a 0.03 m portion of ∅4 shrinkable sleeve (4) at each end of the cable, covering the flexible PVC coating and partially the tubular rivet (3). The shrinkable sleeve will confer protection to the fibers when handling the patch cord, because it is an area more susceptible to breakage; alternatively to the use of a shrinkable sleeve it can be used liquid silicone molded to the configuration of terminals versus fiber cable (1);
- Retracting the sleeve (4) with the help of a hot air station;
- Application of a 0.05m portion of ∅9 shrinkable sleeve (5) at the end of the cable (1) with the rivet of 0.028mm (2), placing afterwards a small drop of adhesive (cyanoacrylate) on the rivet and making it slide about the optic connector (6) till the head of the rivet internally abuts. The application of cyanoacrylate adhesive serves to ensure that the terminal does not move within the optic connector and consequently reduce the light transmission capacity of the patch cord;

- Adjusting the ∅9 shrinkable sleeve (5) on the collar of the connector and retract it, thus adjusting the cable (1) to the connector;
- Placing the applicable optical device at the opposite end of the cable;
- The thermal filter is a part of the cable itself in the case of cables currently available in the market but, for improving its longevity, the thermal filter (18) is here applied in the coupling, for instance a Storz type (17), thus decentralizing the overheating factor to the coupling and preventing early destruction of the fibers when connected to a light source;
- The coupling includes the thermal filter (18) followed by an O-ring (19) which prevents the filter from rolling out of the coupling. Since there are different types/brands of light sources, the couplings are supplied separately from the patch cord, thus allowing the end user to select and apply the coupling that best matches their use.

### G. Packing of illuminator and package sealing:

- Preparing the thermal sealer;
- Turning on the thermal sealer by turning the on/off button located on the left side of the machine;
- Confirming availability of sealing film to seal the packages;
- Turning on the thermal sealer system by pressing the "on" button;
- Waiting until the system reaches the desired temperature;
- Pressing the two green buttons simultaneously and holding under pressure until the sealant mold enters the sealing area;
- Waiting for the sealing process to take place;
- Removing the packages already sealed after the sealing cycle ends;
- Placing the cable (1) of the illuminator in the thermoformed package, being the applicable optical device housed in the "box" of the package periphery and the optic connector in the inner "box" of the package;
- Placing the retaining clips on respective latches after packaging the cable (1) in the package;
- Placing the package in the thermal sealer tray with the package "head" oriented upward;
- Waiting the thermal sealer reaches the desired temperature (122°C);
- Starting the sealing;
- Pressing the two green buttons simultaneously and holding under pressure until the sealant mold enters the sealing area;
- Waiting for the sealing process to take place;
- Removing the packages already sealed after the sealing cycle ends;
- Printing the labels with information relating to the lot;
- Application of the label on the sealing paper, in the respective placing area;
- Placing the packages in card boxes of 5 units each.

### H. Finalization of the lot:

The lot number is assigned to the quantity produced in a production cycle, so it may have different quantities. As such, the printed form that records all the history of the lot accompanies each lot of each production cycle.

The final lot for sending to sterilization is composed of the volume that occupies a Euro-pallet. The 5 units boxes are packed in corrugated boxes (14×5 units). The Euro-pallets are lined with plastic wrap and send to the sterilizer.

The product obtained by present invention method is a patch cord with the following technical features:
- Terminals;
- Connection or connector;
- 19 PMMA optical fibers with 0.0005m diameter, coated with a 0.0025m flexible crystal PVC sleeve (0.0025m refers to the inner diameter of the fiber, the outer diameter being ±0.0045m) using an ultraviolet curing liquid adhesive;

- The ends of the fiber pass each rivet in an average length of 0.01m;
- Ultraviolet curing adhesive on each end of the fiber;
- 0.03m of shrinkable sleeve (4) at each end of the cable, covering the flexible PVC coating and partially the tubular rivet (3);
- The cable end with the 0.028mm rivet (2) presents a 0.05m portion of shrinkable sleeve (5) and adhesive over the rivet;
- The opposite end of the cable presents the applicable optical device;
- The coupling includes the thermal filter (18) followed by an O-ring (19).

The previous description for a preferred embodiment of the invention is presented for purposes of illustration and description and is not intended to limit the invention to the form disclosed, being possible modifications and variations in accordance with the above description.

For instance, the polishing method may be replaced by other methods whose results reach or exceed the levels of light transfer of the current method, such as an automatic equipment existing in the market constituted by a unit that comprises three distinct stages: polishing, cleaning and inspection, where the polishing and cleaning are subdivided into three substations, progressively maximizing the polishing and cleaning levels, respectively, and the visual inspection gives greater accuracy in cable quality.

## Claims

1. Method for production of local illumination patch cords with the following steps:
a) Cleaning and drying of tubular rivets (2) (3) and optic connector (6);
b) Cutting and assembling the tubular rivets (2) (3) in a fiber;
c) Application of an ultraviolet curing adhesive;
d) Ultraviolet exposure;
wherein it further comprises:
e) Polishing the fibers;
f) Assembling the optic connector (6) and an optical device; **characterized by**
g) Application of a thermal filter (18) in a coupling (17), followed by an O-ring (19); and
h) said coupling (17) being supplied separately from the patch cord, allowing accommodation of the patch cord in different types of light sources.

2. Method according to claim 1, **characterized in that** the ultraviolet exposure process is performed with a UV lamp (7) of about 1500W.

3. Method according to claim 1, **characterized in that** a rectification of the ends is made in a fine-grained grinding wheel (11) followed by sliding the support on a guide bar (14) to the felt buffing wheel (12) with soap, and respective polishing for 30 seconds.

4. Method according to claim 1, **characterized in that** the assembling procedure of the optic connector (6) and of the applicable optical device comprises the following steps:
a) Application of a 0.03m portion of shrinkable sleeve (4) at each end of the cable, covering the flexible PVC coating and partially the tubular rivet (3);
b) Retracting the sleeve (4) with the help of a hot air station;
c) Placing of a 0.05m portion of shrinkable sleeve (5) at the end of the cable presenting the rivet (2) ;
d) Placing of an adhesive on the rivet (2) and respective sliding about the optic connector (6) till the head of the rivet internally abuts;
e) Adjusting the shrinkable sleeve (5) on the collar of the connector and retracting it, thus adjusting the cable to the connector;
f) Placing the applicable optical device at the opposite end of the cable;
g) Application of the thermal filter (18) in the coupling (17) followed by an O-ring (19).

5. Patch cord obtained by the method of claims 1 to 4, and comprising:
a) Terminals;
b) Connection or connector;
c) 19 PMMA optical fibers with 0.0005m diameter, coated with a 0.0025m flexible crystal PVC sleeve using an ultraviolet curing liquid adhesive;
d) The ends of the fibers pass a rivet in an average length of 0.01m;
e) Ultraviolet curing adhesive on each end of the fibers
f) 0.03m of shrinkable sleeve (4) at each end of the cable, covering the flexible PVC coating and partially the tubular rivets;
g) 0.05m portion of shrinkable sleeve (5) and adhesive over the rivet at the end of the cable with the rivet of 0.028mm (2);
h) an optical device placed at the opposite end of the cable;
i) a coupling (17) including a thermal filter (18) followed by an O-ring (19), said coupling (17) being supplied separately from the patch cord, allowing accommodation of the patch cord in different types of light sources.

## Patentansprüche

1. Verfahren zur Herstellung von lokalen Verbindungskabeln für Beleuchtung mit folgenden Schritten:
a) Reinigung und Trocknung von Rohrnieten (2) (3) und Lichtwellenleiterstecker (6);
b) Schneiden und Zusammenfügen der Rohrnieten (2) (3) in einer Fiber;
c) Einsatz von einem ultravioletten härtenden Klebstoff;
d) Ultraviolette Bestrahlung;
worin es weiterhin folgendes umfasst:
e) Polieren der Fasern;
f) Zusammenfügen des Lichtwellenleitersteckers (6) und einer optischen Vorrichtung; **dadurch gekennzeichnet, dass**
g) Einsatz eines thermischen Filters (18) in einer Kupplung (17), gefolgt von einem O-Ring (19); und
h) die besagte Kupplung (17) wird getrennt von dem Verbindungskabel geliefert, und erlaubt so die Aufnahme von dem Verbindungskabel in verschiedenen Arten von Lichtquellen.

2. Verfahren nach Anspruch 1, **dadurch gekennzeichnet, dass** der ultraviolette Strahlungprozess durch eine UV-Lampe (7) von etwa 1500W ausgeführt wird.

3. Verfahren nach Anspruch 1, **dadurch gekennzeichnet, dass** ein Schleifen der Enden in einer feinkörnigen Schleifscheibe (11) erfolgt, daraufhin gleitet die Stütze mit Seife auf einer Führungsschiene (14) zu der Filzschwabbelscheibe (12), und jeweiliges Polieren für 30 Sekunden.

4. Verfahren nach Anspruch 1, **dadurch gekennzeichnet, dass** der Zusammenfügungsprozess des Lichtwellenleitersteckers (6) und der einsatzbaren optischen Vorrichtung folgende Schritte umfasst:
a) Einsatz von einem 0,03m Teil von schrumpfbarer Hülse (4) an jedem Ende des Kabels, so wird die PVC-Beschichtung und zum Teil die Rohrniete (3) bedeckt;
b) Zurückziehen der Hülse (4) mit der Hilfe einer Heißluftstation;
c) Platzieren von einem 0,05m Teil von schrumpfbarer Hülse (5) am Ende des Kabels, das die Niete (2) aufweist;
d) Platzieren eines Klebstoffs auf der Niete (2) und jeweiliges Gleiten um des Lichtwellenleitersteckers (6) bis der Kopf der Niete intern anstoßt;
e) Anpassung der schrumpfbaren Hülse (5) an den Kragen des Steckers und der diesen zurückzieht, so das Kabel an den Stecker anpasst;
f) Platzieren der einsatzbaren optischen Vorrichtung an dem gegenüberliegenden Ende des Kabels;
g) Einsatz des thermischen Filters (18) in der Kupplung (17), gefolgt von einem O-Ring (19).

5. Verbindungskabel erhalten durch das Verfahren von Ansprüchen 1 bis 4, welches folgendes umfasst:
a) Terminals;
b) Verbindung oder Stecker;
c) 19 PMMA optische Fasern mit 0,0005m Durchmesser, mit einer 0,0025m kristallen biegsamen PVC-Hülse beschichtet, mit Hilfe eines ultravioletten härtenden flüssigen Klebstoffs;
d) Die Enden der Fasern überqueren einen Niete in einer Durchschnittslänge von 0,01m;
e) Ultravioletter härtender Klebstoff an jedem Ende der Fasern;
f) 0.03m von schrumpfbarer Hülse (4) an jedem Ende des Kabels, welche die biegsame PVC-Beschichtung und zum Teil die Rohrnieten bedeckt;
g) 0.05m Teil von schrumpfbarer Hülse (5) und Klebstoff über der Niete am Ende des Kabel mit der Niete von 0.028mm (2);
h) eine optische Vorrichtung, die am gegenüberliegendem Ende des Kabels platziert wird;
i) eine Kupplung (17) mit einem thermischen Filter (18), gefolgt von einem O-Ring (19), die besagte Kupplung (17) wird getrennt von dem Verbindungskabel geliefert, und erlaubt so die Aufnahme von dem Verbindungskabel in verschiedenen Arten von Lichtquellen.

## Revendications

1. Procédé de production de cordons de raccordement d'éclairage local comportant les étapes suivantes:
a) Nettoyage et séchage des rivets tubulaires (2) (3) et du connecteur optique (6);
b) Découpe et assemblage des rivets tubulaires (2) (3) dans une fibre;
c) Application d'un adhésif durcissant aux ultraviolets;
d) Exposition aux ultraviolets;
dans lequel il comprend en outre:
e) Le polissage des fibres;
f) L'assemblage du connecteur optique (6) et d'un dispositif optique; **caractérisé par**
g) L'application d'un filtre thermique (18) dans un couplage (17), suivi d'un joint torique (19); et
h) ledit couplage (17) étant fourni séparément du cordon de raccordement, ce qui permet de loger le cordon de raccordement dans différents types de sources lumineuses.

2. Procédé selon la revendication 1, **caractérisée en ce que** le processus d'exposition aux ultraviolets est effectué avec une lampe UV (7) d'environ 1500W.

3. Procédé selon la revendication 1, **caractérisé en ce que** la rectification des extrémités est effectuée dans une meule à grain fin (11), suivie par le glissement du support sur une barre de guidage (14) vers la meule de polissage de feutre (12) avec du savon, et le polissage respectif pendant 30 secondes.

4. Procédé selon la revendication 1, **caractérisé en ce que** la procédure d'assemblage du connecteur optique (6) et du dispositif optique applicable comprend les étapes suivantes:
a) Application d'une portion de 0,03m de manchon rétractable (4) à chaque extrémité du câble, recouvrant le revêtement souple en PVC et partiellement le rivet tubulaire (3);
b) Rétraction du manchon (4) à l'aide d'une station à air chaud;
c) Mise en place d'une portion de 0,05m de manchon rétractable (5) à l'extrémité du câble présentant le rivet (2);
d) Mise en place d'un adhésif sur le rivet (2) et glissement respectif autour du connecteur optique (6) jusqu'à ce que la tête du rivet vienne en butée en interne;
e) Ajustement du manchon rétractable (5) sur le collier du connecteur et rétraction de celui-ci, ce qui permet d'ajuster le câble au connecteur;
f) Mise en place du dispositif optique applicable à l'extrémité opposée du câble;
g) Application du filtre thermique (18) dans le couplage (17) suivi d'un joint torique (19).

5. Cordon de raccordement obtenu par la procédé des revendications 1 à 4, et comprenant:
a) Des bornes;
b) Une connexion ou un connecteur;
c) 19 fibres optiques en PMMA de 0,0005m de diamètre, revêtues d'un manchon en PVC cristal flexible de 0,0025m en utilisant un adhésif liquide durcissant aux ultraviolets;
d) Les extrémités de la fibre passent un rivet d'une longueur moyenne de 0,01m;
e) Adhésif durcissant aux ultraviolets à chaque extrémité de la fibre;
f) 0,03m de manchon rétractable (4) à chaque extrémité du câble, recouvrant le revêtement souple en PVC et partiellement les rivets tubulaires;
g) une portion de 0,05m de manchon rétractable (5) et de l'adhésif sur le rivet à l'extrémité du câble comportant le rivet de 0,028mm (2);
h) un dispositif optique placé à l'extrémité opposée du câble;
i) un couplage comprenant un filtre thermique (18) suivi d'un joint torique (19), ledit couplage (17) étant fourni séparément du cordon de raccordement, ce qui permet de loger le cordon de raccordement dans différents types de sources lumineuses.
